(19) Europäisches Patentamt

European Patent Office

Office européen des brevets

(11) Veröffentlichungsnummer: **0 069 833**
**B1**

(12) # EUROPÄISCHE PATENTSCHRIFT

(45) Veröffentlichungstag der Patentschrift:
10.07.85

(21) Anmeldenummer: 82103463.4

(22) Anmeldetag: 23.04.82

(51) Int. Cl.⁴: **G 01 N 33/36, G 01 N 9/36**

(54) Verfahren und Vorrichtung zur Bestimmung der Dichte von Faserbündeln.

(30) Priorität: 13.07.81 CH 4573/81

(43) Veröffentlichungstag der Anmeldung:
19.01.83 Patentblatt 83/3

(45) Bekanntmachung des Hinweises auf die Patenterteilung:
10.07.85 Patentblatt 85/28

(84) Benannte Vertragsstaaten:
DE FR GB IT

(56) Entgegenhaltungen:
EP - A - 0 017 673
DE - B - 2 167 255
DE - B - 2 330 972
GB - A - 1 224 123

(73) Patentinhaber: ZELLWEGER USTER AG, Wilstrasse 11,
CH-8610 Uster (CH)

(72) Erfinder: Felix, Ernst, Bahnstrasse 35, CH-8610 Uster
(CH)

(74) Vertreter: Dipl.-Phys.Dr. Manitz Dipl.-Ing. Finsterwald
Dipl.-Ing. Grämkow Dipl.-Chem.Dr. Heyn
Dipl.-Phys.Rotermund, B.Sc. Morgan
Robert-Koch-Strasse 1, D-8000 München 22 (DE)

## Beschreibung

Die Erfindung betrifft ein Verfahren zur Bestimmung der Dichte eines Faserbündels, bei dem das Faserbündel auf einen definierten Querschnitt gebracht wird. Ferner ist die Erfindung auf eine Vorrichtung zur Durchführung dieses Verfahrens gerichtet.

In der Textilindustrie ist die Bestimmung der Substanzmenge von Fasermengen, insbesondere des Substanzquerschnittes von Faserbändern sehr wichtig. Dem Substanzquerschnitt von Faserbändern kommt in der Weiterverarbeitung erhebliche Bedeutung zu, da dieser die Feinheit der Garne und deren Querschnittsgleichmäßigkeit direkt beeinflußt.

Primär handelt es sich darum, die Substanzmenge einer Fasermenge zu bestimmen, ohne Rücksicht darauf, ob diese sich in ruhendem oder in bewegtem Zustand befindet. Die laufende Messung von Faserbändern unterscheidet sich im Prinzip hiervon nicht, lediglich die Organe zur Führung und Begrenzung der Faserbänder sind entsprechend auszubilden.

Die Gewichtsbestimmung insbesondere an laufenden Faserbändern ist aber direkt praktisch unmöglich. Es sind deshalb schon zahlreiche Lösungsvorschläge aufgezeigt worden, um diesen Substanzquerschnitt indirekt zuverlässig zu messen. Als Beispiele seien genannt:

— die rein mechanische Messung mittels Nutenwalze und Tastrolle, bei der das Faserband durch die Nutenwalze geführt und der Achsabstand zwischen Nutenwalze und Tastrolle als Maß für den Substanzquerschnitt ausgewertet wird. Diese Anordnung ist wegen ihres hohen mechanischen Aufwandes und der begrenzten Frequenz infolge großer mechanisch zu bewegender Massen nur wenig verbreitet.

— Optische Meßorgane sind ebenfalls bekannt, die das Absorptions- oder Reflexionsvermögen der Faserbänder ausnutzen. Auch diese Meßsysteme weisen einen hohen Aufwand an elektrischen und optischen Mitteln auf, außerdem ist die Abhängigkeit der Lichtabsorption — oder — Reflexion von der Farbe des Fasermaterials der Zuverlässigkeit solcher Systeme abträglich.

— Akustische Meßorgane sind ebenfalls bekannt. Bei diesen wird die Dämpfung von Luftschallwellen, die ein Faserband durchdringen, gemessen. Die Genauigkeit vermag aber nicht zu befriedigen.

— Als weiterer Vertreter in der Reihe der Meßorgane für den Substanzquerschnitt von Faserbändern sei noch das sogenannte aktiv-pneumatische Meßorgan genannt. Dieses besteht im wesentlichen aus einem Trichter, an den seitlich eine Druckmeßvorrichtung angeschlossen ist. Beim Durchlauf des Faserbandes durch den Trichter treten an diesem seitlichen Anschluß Druckschwankungen auf, die dem jeweiligen Querschnitt entsprechen und in geeigneten Wandlern in äquivalente Signale umgeformt werden. Dieses aktiv-pneumatische Meßorgan ist an sich einfach im Aufbau; jedoch weist es als Nachteile eine Abhängigkeit des Meßwertes sowohl von der Faserfeinheit (Micronair-Wert), als auch von der Durchlaufgeschwindigkeit auf.

Aufgabe der Erfindung ist es, die Nachteile dieser bekannten Meßsysteme zu beseitigen.

Dies wird dadurch erreicht, daß eine dem Elastizitätsmodul entsprechende Größe gemessen wird und daß davon die Dichte des Faserbündels abgeleitet wird.

Eine Vorrichtung zur Bestimmung der Dichte eines Faserbündels, bei der ein definierter Querschnitt in Form einer den Querschnitt des Faserbündels definierenden Führung nach Art eines Rohres, Trichters oder einer Trompete vorgesehen ist, zeichnet sich nach der Erfindung dadurch aus, daß der Führung Vorrichtungen zur Messung einer dem Elastizitätsmodul entsprechenden Größe und zur Ableitung der Dichte des Faserbündels aus dieser Größe zugeordnet sind.

Weitere Ausgestaltungen der Erfindung sind in den Unteransprüchen angegeben.

Anhand der Beschreibung und der Figuren wird die Erfindung näher erläutert. Dabei zeigt

Fig. 1 schematisch die Basis für den Grundgedanken des Meßprinzips;

Fig. 2 ein Kraft-Dehnungs-Diagramm;

Fig. 3 ein Schwingungssystem aus Masse und Feder;

Fig. 4 eine Meßvorrichtung für die Bestimmung der Übertragungsfunktion im Meßmaterial;

Fig. 5 ein Impulsdiagramm;

Fig. 6 eine erste Variante des Meßverfahrens nach Fig. 4;

Fig. 7 eine weitere Variante des Meßverfahrens nach Fig. 4;

Fig. 8 einen Querschnitt durch eine erste Form eines genau definierten Raumes;

Fig. 9 einen Längenschnitt des Raumes nach Fig. 8;

Fig. 10 einen Querschnitt durch eine andere Form eines genau definierten Raumes;

Fig. 11 eine konstruktive Ausführung einer Meßvorrichtung im Längsschnitt;

Fig. 12 ein konstruktives Detail eines Gebers oder Empfängers im Längsschnitt.

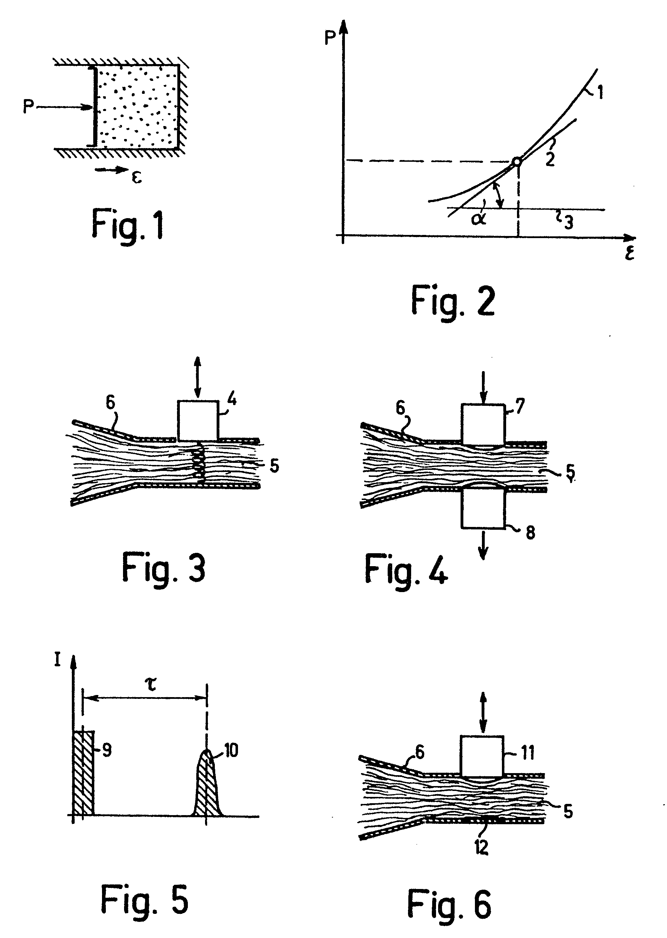

Der Erfindung liegt vorerst folgende Beobachtung zugrunde: Textile Fasermengen sind in der Regel ein Gebilde aus Fasern mit sehr großen Luftzwischenräumen. Selbst bei mäßiger Pressung ist der reine Materialanteil (Substanzmenge) der Fasern relativ klein. So beträgt er beispielsweise an den Trichtern von Karden nur 10—20%. Wenn nun eine lockere Fasermenge gepreßt wird (Fig. 1), ergibt sich ein bestimmter Verlauf der Kraft-Dehnungskurve (Fig. 2). Da an die Fasermenge keine Zugkräfte, sondern nur Druckkräfte (Pressungen) angelegt werden können, entspricht der Dehnung eine Pressung; dies zum Unterschied zu den allgemein üblichen Kraft-Dehnungs-Diagrammen fester Körper. Gemäß der graphischen Darstellung in Fig. 2 ist die Abszissenrichtung die Dehnung $\varepsilon$ (Pressung), und die Kraft P in Ordinatenrichtung als Kurvenzug 1 gezeigt. Der Elastizitätsmodul E kann in bekannter Weise in jedem Punkt durch den Tangens des Winkels $\alpha$, den eine an die Kurve 1 gelegte Tangente 2 mit der Horizontalen 3 einschließt, ausgedrückt werden:

$$E = \operatorname{tg} \alpha \qquad (1)$$

Beobachtungen haben ergeben, daß einer bestimmten Dichte der Fasermenge eine bestimmte Kraft und damit ein bestimmter Elastizitätsmodul entspricht. Unter der Dichte der Fasermenge ist hierbei der Mittelwert aus dem Gemisch von Fasermaterial und der Luft in dessen Zwischenräumen des Fasermaterials zu verstehen. Daher gilt: Reiner Substanzquerschnitt $Q_0$ des Faserbandes ist gleich dem Querschnitt des Faserbandes x Füllfaktor F.

Die Veränderung der Kraft bzw. des Elastizitätsmoduls in Funktion der Dichte ist sehr ausgeprägt. Hieraus entspringt nun der erfinderische Gedanke, daß an irgendeiner Stelle, an welcher die gesamte Fasermenge einen definierten Raum oder Querschnitt aufweist, die Preßkraft bzw. der Elastizitätsmodul direkt oder indirekt bestimmt und daraus auf die Dichte geschlossen wird. Aus der Dichte und dem genannten Raum oder Querschnitt kann hernach die reine Materialmenge bestimmt werden.

Es ist allerdings darauf hinzuweisen, daß die Kraft bzw. der Elastizitätsmodul in Funktion der Pressung bei mehreren Meßvorgängen der gleichen Fasermenge in der Regel ungleiche Resultate ergibt. Vor allem bei der ersten Pressung der losen Fasermenge ist ein höherer Kraftbedarf notwendig als bei den nächstfolgenden. Für die erfindungsgemäße Bestimmung der Substanzmengen in Fasermengen ist es daher vorteilhaft, nur die Resultate der ersten Pressung oder nur die Resultate wiederholter Pressung auszuwerten. Besonders vorteilhaft ist es dabei, die Bestimmung der Pressung in Rohren oder im Bereich der Trichter (Trompeten) von Karden, Strecken oder dergleichen vorzunehmen, weil dort die Fasermengen betriebsmäßig und in der Regel kontinuierlich von einem losen Zustand in eine Verengung geführt werden und somit eigentlich immer die erste Pressung stattfindet. Ein weiterer Vorteil solcher Meßstellen ist darin zu sehen, daß der äußere Faserbandquerschnitt genau definiert werden kann. Nachdem dieser Anwendungsfall wohl den häufigsten Einsatz eines Meßorgans nach dem erfindungsgemäßen Verfahren ist, sollen in den nächsten Abschnitten vorwiegend Meßverfahren zur Bestimmung der Substanzmenge von Faserbändern behandelt werden, die durch einen bestimmten Querschnitt geführt werden. Grundsätzlich sind die Überlegungen aber auch gültig für Messungen an ruhendem Material in einem genau definierten Raum.

Die direkte Bestimmung der Kraft für die Pressung im laufenden Faserband ist aber aus praktischen Gründen schwierig. Aus diesem Grund ist die Bestimmung des Elastizitätsmoduls vorteilhafter. Hieraus kann dann auf die Dichte geschlossen werden. Die Realisierung dieses Erfindungsgedankens kann durch verschiedene Varianten erreicht werden:

### Die Fasermenge als Feder

Bekanntlich setzt sich ein einfaches Schwingungssystem aus einer Masse und einer Feder zusammen. Aus Masse und Federkonstanten läßt sich die Resonanzfrequenz bestimmen. Wird im vorliegenden Fall gemäß Fig. 3 ein Körper 4 mit einer vorgegebenen Masse an das Faserband 5 (= Feder) seitlich angedrückt, so ergibt sich ebenfalls ein Schwingungssystem. Die Eigenfrequenz bestimmt sich aus der Masse des Körpers 4 und der Federkonstanten (Elastizitätsmodul E) des Faserbandes. Eventuell müssen noch Federkonstanten und Massen der Befestigungsorgane der Masse mitberücksichtigt werden. Das Faserband 5 kann nun auch in einem Rohr oder Trichter 6 bewegt werden, wobei es unter der Masse 4 durchgleitet. Verändert sich dabei die Dichte des Faserbandes 5, so ändern sich auch der Elastizitätsmodul E bzw. die Federkonstante und damit die Resonanzfrequenz. Daraus kann die Dichte des Faserbandes bestimmt werden. Die Anregung eines Schwingungssystems, das aus einer Masse und einer Feder besteht, ist eine an sich bekannte Maßnahme und deshalb hier nicht weiter erläutert.

### Bestimmung der Übertragungseigenschaften

Jedes feste, flüssige oder gasförmige Medium besitzt eine bestimmte Fortpflanzungsgeschwindigkeit c eines Druckes oder einer Auslenkung mit einer gewissen Dämpfung D (Übertragungsfunktion). Die Fortpflanzungsgeschwindigkeit wird oft auch mit Schallgeschwindigkeit bezeichnet. Die Fortpflanzungsgeschwindigkeit c kann berechnet werden aus

$$c = \sqrt{\frac{E}{D}},$$

wobei

E = Elastizitätsmodul
D = Dichte.

Diese Gesetze sind aus der allgemeinen Physik bekannt.

In der vorliegenden Erfindung wird davon ausgegangen, daß bei Fasermengen eine gewisse Analogie zum Übertragungsverhalten in homogenen, festen, flüssigen oder gasförmigen Körpern vorliegt. Während der Übertragungsvorgang bei homogenen Körpern jedoch im molekularen Bereich (von Molekül zu Molekül) stattfindet, erfolgt diese bei Fasermengen im makroskopischen Bereich (von Faser zu Faser). Eine weitere Abweichung der Analogie zu homogenen Körpern besteht darin, daß bei solchen das Übertragungsverhalten durch nahezu feste Materialkonstanten bestimmt ist. Im vorliegenden Fall ist aber — wie beschrieben — die Dichte und vor allem der Elastizitätsmodul stark abhängig von der Pressung und umgekehrt. Dies wird in der vorliegenden Erfindung ausgenützt, indem sich aus der Bestimmung der Übertragungsfunktion, vorzugsweise aus der Fortpflanzungsgeschwindigkeit die Dichte der Fasermenge und damit z. B. auch deren Substanzquerschnitt bestimmen läßt.

Fig. 4 zeigt das Verfahrensprinzip für die Bestimmung der Fortpflanzungsgeschwindigkeit und/oder der Dämpfung an einem stehenden oder laufenden Faserband. 6 stellt ein Rohr oder einen Trichter im Querschnitt dar; 7 ist ein Geber, der in der Lage ist, mechanische Deformationen zu erzeugen und an das Faserband 5 weiterzugeben. Besonders zweckmäßig hierzu sind Piezokristalle. Wird nun beispielsweise ein Impuls vom Geber 7 an das Faserband 5 übertragen, so pflanzt sich dieser im Faserband 5 mit der Fortpflanzungsgeschwindigkeit c und einer gewissen Dämpfung d fort und wird nach einer bestimmten Zeit $\tau$ von einem Empfänger 8 empfangen. Auch dieser Empfänger 8 kann als Piezokristall ausgebildet sein.

Dieser Laufzeit $\tau$ (Fig. 5) entspricht nun eine genau bestimmte Dichte D des Faserbandes 5. Ist das Faserband locker, ist die Fortpflanzungsgeschwindigkeit klein und die Dämpfung groß. Wird das Faserband dichter, steigt auch die Fortpflanzungsgeschwindigkeit und die Dämpfung sinkt. Demzufolge kann also aus der Fortpflanzungsgeschwindigkeit und/oder der Dämpfung die Faserdichte D und damit der reine Substanzquerschnitt $Q_0$ des Faserbandes bestimmt werden (Fig. 5).

Der Empfangsimpuls 10 ist zeitlich um die Laufzeit $\tau$ vom Geberimpuls 9 entfernt.

Als Variante der Anordnung gemäß Fig. 4 ist auch eine solche nach Fig. 6 möglich. Dabei ist ein Wandler 11 sowohl als Geber als auch Empfänger eingesetzt, und diesem gegenüber ist der Trichter so ausgebildet, daß er als Reflektor 12 wirkt. Der Impuls durchläuft somit das Faserband 5 zweimal, d. h. hin und zurück, und erscheint somit nach 2 $\tau$ am Wandler 11 — wiederum mit einer bestimmten Dämpfung — als Empfangssignal.

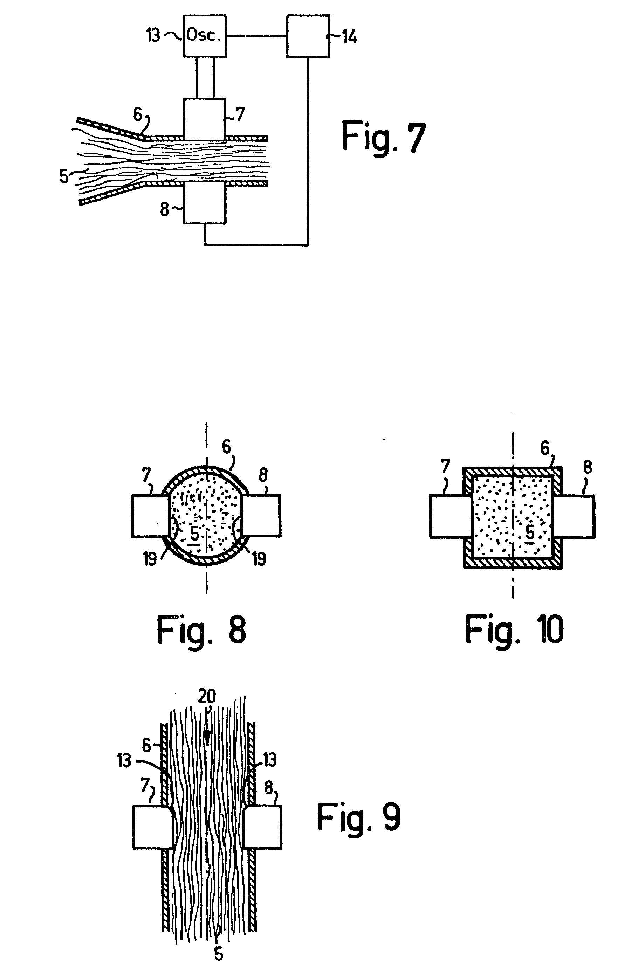

Äquivalent der Messung der Laufzeit $\tau$ ist auch die Ausnützung der Resonanzfrequenz $f_0$ eines durch Rückkopplung gebildeten Schwingungssystems (Fig. 7).

Da die Resonanzfrequenz umgekehrt proportional der Laufzeit $\tau$ ist, kann auch auf diese Weise die Dichte D des Faserbandes 5 ermittelt werden. Dabei wird das Schwingungssystem durch eine geeignete, an sich bekannte Rückkopplung 14 zwischen Geber 7 und Empfänger 8 im Resonanzbereich angeregt. Beispielsweise wird für die Rückkopplung das Empfängersignal auf einen sogenannten »phase-locked-loop« (PLL) geleitet, der aus der Phasenlage am Empfänger 8 den Geber 7 in seiner Frequenz steuert.

Dabei ergibt sich ein Resonanzsystem nicht nur durch die Rückkopplung, sondern auch innerhalb der Fasermenge. Denn dort bestehen bereits Resonanzen, die durch die genannte Rückkopplung lediglich noch angeregt werden. Ihre Resonanzfrequenzen sind umgekehrt proportional zur zweifachen Laufzeit.

Dieses Verfahren ist besonders vorteilhaft an laufenden Faserbändern, da laufende Faserbänder durch ihre Bewegung am Empfänger ein starkes Rauschen als Störsignal erzeugen. Mit Hilfe von Resonanzsystemen lassen sich diese Störsignale weitgehend eliminieren.

Weitere Resonanzfrequenzen liegen auch auf Harmonischen der Grundschwingung.

Meßorgane nach dem erfindungsgemäßen Verfahren werden vorzugsweise auf Textilmaschinen eingesetzt, wo der Materialfluß mehr oder weniger kontinuierlich ist und die Fasermenge einen genau definierten Querschnitt durchläuft, also beispielsweise in Trichtern (Trompeten) von Karden, Kämmaschinen, Strekken und dergleichen. Sie können dort zur Bestimmung des reinen Substanzquerschnittes und damit als Überwachungsorgane eingesetzt werden, oder sie können zur Steuerung und/oder Regelung der Fasermenge eingesetzt werden.

Fig. 8 bis 10 zeigen Beispiele von Bandführungsorganen (z. B. Trichtern). In Fig. 8 ist der Querschnitt nahezu kreisförmig; Geber 7 und Empfänger 8 sind seitlich in die Wandung des von Faserband 5 gefüllten Trichters 6 eingesetzt. Sie sind vorzugsweise nicht der Kreisform angepaßt, sondern planparallel, damit die Distanzen zwischen den aktiven Geber- und Empfängerflächen 19 gleich sind.

Fig. 9 zeigt den entsprechenden Längsschnitt. Geber 7 und Empfänger 8 reichen leicht in den Raum hinein. In Laufrichtung 20 des Faserbandes 5 angebrachte Abschrägungen 13 an Geber 7 und Empfänger 8 erleichtern den Durchlauf des Faserbandes 5.

Fig. 10 zeigt einen rechteckigen Querschnitt eines Trichters 6 mit Geber 7 und Empfänger 8.

Es sind aber auch andere Querschnittsformen — beispielsweise oval oder mehreckig — möglich.

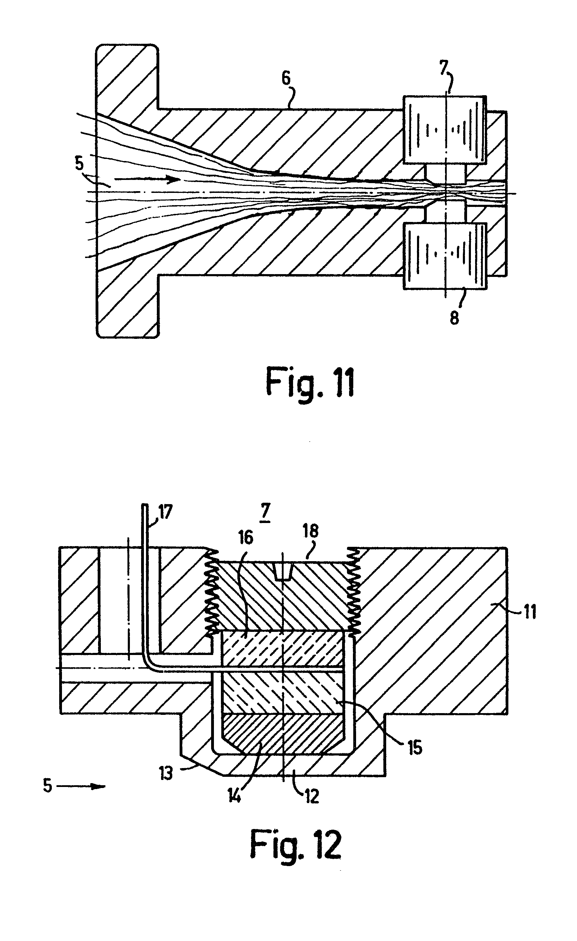

Fig. 11 zeigt ein Konstruktionsbeispiel eines Trichters, wie er beispielsweise in einer Karde verwendet wird. Er ist, abgesehen vom eingesetzten Geber 7 und eingesetzten Empfänger 8 in seiner Konstruktion im Prinzip genau gleich wie die meistens bereits verwendeten Trichter an Karden. 5 ist das durchlaufende Faserband.

In einem solchen Trichter können nun nach dem erfindungsgemäßen Verfahren Impulse vom Geber 7 durch das Faserband 5 auf den Empfänger 8 gelangen. Da derart der äußere Querschnitt des Faserbandes 5 genau definiert ist, sind auch die Dichte und damit der Elastizitätsmodul vorgegeben. Somit kann aus dem Übertragungsverhalten der reine Substanzquerschnitt des Faserbandes hergeleitet werden.

Fig. 12 zeigt als Beispiel den Geber 7 im Schnitt. 11 ist der äußere Körper, wie er in Fig. 11 sichtbar ist. Dieser Körper ist derart bearbeitet, daß zur Fassung und zum Schutze des Piezokristalls gegen das Band 5 hin eine Membrane 12 gebildet wird. Die Abschrägung 13 gewährleistet einen guten Durchlauf des Faserbandes in Pfeilrichtung. Ein Stempel 14 konzentriert die Kraft des Piezokristalls auf die Mitte der Membrane 12. An die beiden Piezokristalle 15 und 16 kann über die Leitung 17 eine elektrische Spannung angelegt und damit eine Deformation erzeugt werden. Die Schraube 18 dient zur leichten Pressung. Elektrisch gesehen ist darauf zu achten, daß die Polarität der Piezokristalle 15 und 16 einerseits gegen die Leitung 17 gleich ist und andererseits gegen die Masse gleich ist. Die Spannung wird zwischen Leitung 17 und Masse angelegt, wobei die Masse aus dem Körper 11, der Schraube 18 und dem Stempel 14 besteht. Diese Konstruktion mit Stempel und Membran ist einer solchen mit direkter Berührung von Faserband 5 und Piezokristall 15 bzw. 16 vorzuziehen.

Die genau gleiche Konstruktion kann auch als Empfänger 8 verwendet werden; hierbei kann zwischen Masse und Leitung 17 das Empfangssignal abgenommen werden.

Das erfindungsgemäße Verfahren bzw. die entsprechende Vorrichtung hat, wie insbesondere das Beispiel der Fig. 11 zeigt, den großen Vorteil, daß ein breites vorhandenes Maschinenelement, nämlich der Trichter 6, als Meßorgan abgewandelt werden kann. Somit treten keine Konstruktionsprobleme auf beim Einbau eines Meßorgans zur Bestimmung des Substanzquerschnittes von Faserbändern.

Zum Problem der Ausnützung des Elastizitätsmoduls zur Bestimmung der Dichte einer Fasermenge ist noch darauf hinzuweisen, daß der Elastizitätsmodul bei einer statischen Messung nicht unbedingt gleich groß ist wie derjenige bei einer dynamischen Messung. Für das Prinzip der Bestimmung der Dichte von Fasermengen spielt dies jedoch keine Rolle.

## Patentansprüche

1. Verfahren zur Bestimmung der Dichte eines Faserbündels, bei dem das Faserbündel auf einen definierten Querschnitt gebracht wird, dadurch gekennzeichnet, daß eine dem Elastizitätsmodul entsprechende Größe gemessen wird und daß davon die Dichte des Faserbündels abgeleitet wird.

2. Verfahren nach Anspruch 1, dadurch gekennzeichnet, daß das Übertragungsverhalten des Faserbündels gegenüber mechanischen Druckänderungen gemessen wird.

3. Verfahren nach den Ansprüchen 1 und 2, dadurch gekennzeichnet, daß die Fortpflanzungsgeschwindigkeit mechanischer Druckänderungen bestimmt wird.

4. Verfahren nach den Ansprüchen 1 und 2, dadurch gekennzeichnet, daß mittels mindestens eines querschnittsbegrenzenden Gebers (7) Druckänderungen auf das Faserbündel (5) ausgeübt werden, und daß in mindestens einem querschnittsbegrenzenden Empfänger (8) die Laufzeit ($\tau$) zwischen Aussendung und Empfang der Druckänderungen ermittelt wird.

5. Verfahren nach den Ansprüchen 1 und 2, dadurch gekennzeichnet, daß mittels mindestens eines querschnittsbegrenzenden Gebers (7) Druckänderungen auf das Faserbündel (5) ausgeübt werden, die an der gegenüberliegenden Seite (12) des definierten Querschnitts reflektiert und vom Geber (7) als Druckänderungen empfangen werden.

6. Verfahren nach Anspruch 1, dadurch gekennzeichnet, daß das Faserbündel (5) mit mindestens einer Masse (4) in Verbindung gebracht wird und so ein Schwingungssystem bildet und daß aus der sich einstellenden Resonanzfrequenz (f) dieses Schwingungssystems die Dichte des Faserbündels (5) ermittelt wird.

7. Verfahren nach den Ansprüchen 1, 2, 4 und 5, dadurch gekennzeichnet, daß in dem Faserbündel (5) mindestens eine Resonanz angeregt wird, indem die Anregungsfrequenz nach dem Reziprokwert der Laufzeit oder dem Reziprokwert eines ganzzahligen Vielfachen der doppelten Laufzeit eingestellt wird, wobei aus der sich einstellenden Resonanzfrequenz die Dichte des Faserbündels (5) ermittelt wird.

8. Vorrichtung zur Bestimmung der Dichte eines Faserbündels, bei der ein definierter Querschnitt in Form einer den Querschnitt des Faserbündels (5) definierenden Führung (6) nach Art eines Rohres, Trichters oder einer Trompete vorgesehen ist, zur Durchführung des Verfahrens nach Anspruch 1, dadurch gekennzeichnet, daß der Führung Vorrichtungen zur Messung einer dem Elastizitätsmodul entsprechenden Größe und zur Ableitung der Dichte des Faserbündels (5) aus dieser Größe zugeordnet sind.

9. Vorrichtung nach Anspruch 8, dadurch gekennzeichnet, daß in die Wandung der Führung (6) mindestens ein Geber (7) eingesetzt ist, daß über diesen mindestens einen Geber das Faserbündel (5) Druckänderungen aussetzbar ist, und daß an mindestens einem Empfänger (8) Druckänderungen meßbar sind.

10. Vorrichtung nach den Ansprüchen 8 und 9, dadurch gekennzeichnet, daß der Empfänger (8) dem Geber (7) gegenüberliegend angeordnet ist.

11. Vorrichtung nach den Ansprüchen 8 und 9, dadurch gekennzeichnet, daß Geber (7) und Empfänger (8) identisch sind und mit einer reflektierenden Zone (12) zusammenwirken.

12. Vorrichtung nach Anspruch 8, dadurch gekennzeichnet, daß der Geber (7) von einem Oszillator (13) angeregt ist, und mittels des Empfängers (8) und einer Phasenmeßvorrichtung (14) die Phasenverschiebung zwischen Geber- und Empfangssignal meßbar ist.

13. Vorrichtung nach Anspruch 12, dadurch gekennzeichnet, daß Oszillator (13) und Phasenmeßvorrichtung (14) einen PLL-Schaltkreis bilden.

14. Vorrichtung nach den Ansprüchen 8 bis 13, dadurch gekennzeichnet, daß Geber (7) und Empfänger (8) Piezokristalle (15, 16) als Druckwandler aufweisen.

15. Vorrichtung nach Anspruch 14, dadurch gekennzeichnet, daß die Piezokristalle (15, 16) mit einer Membrane (12) verbunden sind, die die Druckänderungen auf das Faserbündel (5) übertragen.

16. Vorrichtung nach den Ansprüchen 8 bis 11, dadurch gekennzeichnet, daß durch Geber (7) und Empfänger (8) der definierte Querschnitt bestimmt ist und daß die der Einlaufrichtung (20) des Faserbündels (5) zugekehrten Flanken von Geber (7) und Empfänger (8) eine Abschrägung (13) aufweisen.

17. Vorrichtung nach Anspruch 8, dadurch gekennzeichnet, daß vorhandene und/oder zum Einbau in Karden oder Kämmaschinen vorgesehene Trichter (6) als Meßorgane ausgebildet sind.


**Claims**

1. A method of determining the density of a fibre bundle wherein the fibre bundle is brought to a defined cross-section, characterised in that a value corresponding to the modulus of elasticity is measured; and in that the density of the fibre bundle is derived therefrom.

2. A method in accordance with claim 1, characterised in that the transmission behaviour of the fibre bundle with respect to mechanical pressure changes is measured.

3. A method in accordance with the claims 1 and 2, characterised in that the speed of propargation of mechanical pressure changes is determined.

4. A method in accordance with claims 1 and 2, characterised in that the pressure changes are exerted on the fibre bundle (5) by means of at least one cross-section confining transmitter (7); and in that the transit time ($\tau$) between transmission and receipt of the pressure changes is determined in at least one cross-section confining receiver (8).

5. A method in accordance with the claims 1 and 2, characterised in that pressure changes are exerted on the fibre bundle (5) by means of at least one cross-section confining transmitter (7) which are reflected at the opposite side (12) of the defined cross-section and received by the transmitter (7) as pressure changes.

6. A method in accordance with claim 1, characterised in that the fibre bundle (5) is brought into contact with at least one mass (4) so that an oscillatory system is formed; and in that the density of the fibre bundle (5) is determined from the resonant frequency (f) which arises with this oscillatory system.

7. A method in accordance with the claims 1, 2, 4 and 5, characterised in that at least one resonance is excited in the fibre bundle (5) by adjusting the excitation frequency in accordance with the reciprocal value of the transit time or the reciprocal value of an integral multiple of the double transit time, with the density of the fibre bundle (5) being determined from the resonant frequency which arises.

8. Apparatus for determining the density of a fibre bundle wherein a defined cross-section is provided for carrying out the method of claim 1 in the form of a guide (6) in the nature of a tube, funnel or a trumpet which defines the cross-section of the fibre bundle (5), characterised in that means for measuring a value corresponding to the moduls of elasticity and for deriving the density of the fibre bundle (5) from this value are associated with the guide.

9. Apparatus in accordance with claim 8, characterised in that at least one transmitter is inserted into the wall of the guide (6); in that the fibre bundle (5) can be subjected to pressure changes via said at least one transducer; and in that pressure changes are measurable at at least one receiver (8).

10. Apparatus in accordance with the claims 8 and 9, characterised in that the receiver (8) is arranged opposite to the transmitter (7).

11. Apparatus in accordance with the claims 8 and 9, characterised in that the transmitter (7) and the receiver (8) are identical and cooperate with a reflecting zone (12).

12. Apparatus in accordance with claim 8, characterised in that the transmitter (7) is excited by an oscillator (13); and in that the phase displacement between the transmitter signal and the received signal can be measured by means of the receiver (8) and a phase measuring device (14).

13. Apparatus in accordance with claim 12, charcterised in that the oscillator (13) and the phase measuring device (14) form a PLL (phase locked loop) circuit.

14. Apparatus in accordance with the claims 8 to 13, characterised in that the transmitter (7) and the receiver (8) habe piezo crystals (15, 16) as pressure transformers.

15. Apparatus in accordance with claim 14, characterised in that the piezo crystals (15, 16) are connected to a membrane (12) which transmits the pressure changes to the fibre bundle (5).

16. Apparatus in accordance with claims 8 to 11, characterised in that the defined cross-section is determined by the transmitter (7) and receiver (8); and in that the flanks of the transmitter (7) and receiver (8) which face the entry direction (20) of the fibre bundle (5) have a chamfer (13).

17. Apparatus in accordance with claim 8, characterised in that funnels (6) which are present and/or provided for assembly into cards or combing machines are constructed as measuring devices.

## Revendications

1. Procédé pour la détermination de la densité d'un faisceau de fibres dans laquelle on amène le faisceau de fibres à une section définie, caractérisé en ce que l'on mesure une grandeur correspondant au module d'élasticité et qu'on en tire la densité du faisceau de fibres.

2. Procédé selon la revendication 1, caractérisé en ce que l'on mesure le comportement de transmission du faisceau de fibres à l'égard de variations mécaniques de pression.

3. Procédé selon les revendications 1 et 2, caractérisée en ce que l'on détermine la vitesse de propagation de variations mécaniques de pression.

4. Procédé selon les revendications 1 et 2, caractérisé en ce que l'on exerce des variations de pression sur le faisceau de fibres (5) au moyen d'au moins un transmetteur (7) limitant la section et que dans au moins un récepteur (8) limitant la section, on détermine le temps de propagation ($\tau$) entre émission et réception des variations de pression.

5. Procédé les revendications 1 et 2, caractérisé en ce qu'au moyen d'au moins un transmetteur (7) limitant la section, on exerce sur le faisceau de fibres (5) des variations de pression qui sont réfléchies sur le côté opposé (12) de la section définie et sont reçues par le transmetteur (7) en tant que variations de pression.

6. Procédé selon la revendication 1, caractérisé en ce que l'on met le faisceau de fibres (5) en liaison avec au moins une masse (4) et l'on forme ainsi un système oscillant et que l'on détermine la densité du faisceau de fibres (5) en partant de la fréquence de résonance (f) qui s'établit dans ce système oscillant.

7. Procédé selon les revendications 1, 2, 4 et 5, caractérisé en ce que l'on excite dans le faisceau de fibres (5) au moins une résonance en réglant la fréquence d'excitation selon l'inverse du temps de propagation ou l'inverse d'un multiple entier du double du temps de propagation grâce à quoi on détermine la densité du faisceau de fibres (5) d'après la fréquence de résonance qui s'établit.

8. Dispositif pour la détermination de la densité d'un faisceau de fibres, dans lequel est disposée une section définie sous la forme d'un guide (6) définissant la section du faisceau de fibres (5), du genre d'un tube, d'un entonnoir ou d'une trompette, pour la mise en œuvre du procédé selon la revendication 1, caractérisé en ce qu'au guide sont adjoints des dispostifs pour la mesure d'une grandeur correspondant au module d'élasticité et pour la dérivation de la densité du faisceau de fibres (5) en partant de cette grandeur.

9. Dispositif selon la revendication 8, caractérisé en ce que dans la paroi du guide (6) est inséré au moins un transmetteur (7), que par l'intermédiaire de ce ou de ces transmetteurs le faisceau de fibres (5) peut être exposé à des variations de pression et que sur au moins un récepteur (8) des variations de pression sont mesurables.

10. Dispositif selon les revendications 8 et 9, caractérisé en ce que le récepteur (8) est disposé à l'opposé du transmetteur (7).

11. Dispositif selon les revendications 8 et 9, caractérisé en ce que le transmetteur (7) et le récepteur (8) sont identiques et coopèrent avec une zone réfléchissante (12).

12. Dispositif selon la revendication 8, caractérisé en ce que le transmetteur (7) est excité par un oscillateur (13) et qu'au moyen du récepteur (8) et d'un dispositif de mesure de phase (14), on peut mesurer le déphasage entre signaux de transmetteur et de réception.

13. Dispositif selon la revendication 12, caractérisé en ce que l'oscillateur (13) et le dispositif de mesure de phase (14) forment un circuit PLL.

14. Dispositif selon les revendications 8 à 13, caractérisé en ce que le transmetteur (7) et le récepteur (8) présentent des cristaux piézoélectriques (15, 16) comme transducteurs de pression.

15. Dispositif selon la revendication 14, caractérisé en ce que les cristaux piézoélectriques (15, 16) sont reliés à une membrane qui transmet les variations de pression au faisceau de fibres (5).

16. Dispositif selon l'une quelconque des revendications 8 à 11, caractérisé en ce que par le transmetteur (7) et le récepteur (8) est déterminée la section définit et que les flancs du transmetteur (7) et du récepteur (8) qui sont tournés vers la direction d'entrée du faisceau de fibres (5) présentent un biseau (13).

17. Dispositif selon la revendication 8, caractérisé en ce que des entonnoirs (6) existants et/ou prévus pour l'installation dans des cardes ou des peigneuses sont constitués en organes de mesure.

Fig. 1

Fig. 2

Fig. 3

Fig. 4

Fig. 5

Fig. 6

Fig. 7

Fig. 8

Fig. 10

Fig. 9

**Fig. 11**

**Fig. 12**